# EUROPEAN PATENT SPECIFICATION

(11) **EP 2 632 530 B1**
(45) Date of publication and mention of the grant of the patent: **17.01.2018**
(21) Application number: 11836969.3
(22) Date of filing: 25.10.2011
(51) Int. Cl.: A61B 17/32, A61B 17/11, A61B 17/34, A61M 25/04, A61B 17/00, A61M 25/00, A61M 29/00, A61B 17/3211, A61M 25/02, A61B 90/00

(54) **APPARATUS FOR PENETRATING AND ENLARGING ADJACENT TISSUE LAYERS**
VORRICHTUNG ZUM EINDRINGEN IN BENACHBARTE GEWEBESCHICHTEN UND ZU DEREN VERGRÖSSERUNG
APPAREIL DE PÉNÉTRATION ET D'ÉLARGISSEMENT DE COUCHES TISSULAIRES ADJACENTES

(30) Priority: 25.10.2010 US 406500 P; 26.04.2011 US 201161479097 P
(43) Date of publication of application: 04.09.2013
(73) Proprietor: Boston Scientific Scimed, Inc., Maple Grove, MN 55311 (US)
(72) Inventor: LEPULU, Keke, Mountain View CA 94043 (US); PHAN, Hoang, Mountain View CA 94043 (US)
(74) Representative: Vossius & Partner Patentanwälte Rechtsanwälte mbB
(86) International application number: PCT/US2011/057765
(87) International publication number: WO 2012/058244

(56) References cited:
- EP-A1- 0 829 238
- US-A- 3 039 468
- US-A- 5 275 611
- US-A1- 2008 243 151
- US-A1- 2009 281 557
- US-A1- 2010 268 029
- US-A1- 2010 268 029
- US-B1- 6 508 252
- US-B2- 7 115 136

## Description

### BACKGROUND

Field of the Invention. The present invention relates generally to medical methods and apparatus. More particularly, the present invention relates to methods and apparatus for penetrating adjacent tissue layers and enlarging the resulting penetration.
A number of inter and intra-luminal endoscopic procedures require precise placement of anchors or stents. For example, a number of procedures may be performed by entering the gastrointestinal (GI) tract through a first organ or structure, such as the esophagus, stomach, duodenum, small intestine, or large intestine, and delivering the anchor or stent to adjacent organs and lumen or tissue structures such as an adjacent portion of the GI tract, the bile duct, the pancreatic duct, the gallbladder, the pancreas, cysts, pseudocysts, abscesses, and the like. While primarily intended for use in the GI tract, such methods and apparatus can also be used for access to and from portions of the urinary tract, such as the urinary bladder and ureter, the pulmonary tract, such as the trachea and bronchi, and the biliary tract, such as the bile duct and gallbladder, as well.

Intra-ductal stents are commonly used to facilitate the opening of closed vessels for access, drainage or other purposes. Tissue anchors are used to secure adjacent tissues or organs. Inter-luminal tissue anchors, which include a central lumen, are used to facilitate fluid communication between adjacent ducts, organs or lumens. Often, the precise placement of the tissue anchor or stent is necessary, especially when the tissue anchor or stent has well defined anchoring elements at the proximal and/or distal ends, and the device is used to secure adjacent lumens.

When deploying a stent or other tissue anchor between adjacent body lumens, organs, or other structures, it is typically necessary to penetrate both a wall of the first body lumen through which access is established and a wall of a second body lumen which is the target for the procedure. When initially forming such access penetrations, there is a significant risk of leakage from either or both of the access body lumen and the target body lumen. In some procedures, such as those involving transgastric or transduodenal bile duct access, loss of body fluid into surrounding tissues and body cavities can present a substantial risk to the patient. The risk can be exacerbated when it is necessary to not only penetrate the luminal walls to gain initial access, usually with a needle, but to subsequently enlarge or dilate the initial penetration, for example by passing a tapered dilator over the needle used to establish initial access.

Thus, it would be desirable to establish initial luminal wall penetrations and to subsequently dilate said penetrations in order to deploy a stent, anchor, or for other purposes, while minimizing the risk of body fluid leakage. It would be further desirable to provide improved protocols and access tools which are capable of being deployed from endoscopes present in a first body lumen to access adjacent body lumens or cavities while minimizing the risk of leakage. Such access tools and protocols should be compatible with a wide variety of procedures, such as placement of stents or other tissue anchors between adjacent luminal walls, and will preferably reduce or eliminate the need to exchange tools during the access procedure. It would be further desirable if tools and access protocols could be provided which allow for the continuous application of tension on the luminal walls to maintain said walls in close apposition during the stent or anchor placement or other procedure in order to reduce the risk of body fluid loss during most or all stages of the procedure. At least some of these objectives will be met by the inventions described below.

2. Description of the Background Art. Copending, commonly owned U.S. application numbers 12/772,762, filed on May 3, 2010, and 12/790,553, filed on May 28, 2010, describe luminal wall penetrating and dilating systems. U.S. Published Application Nos. 2009/0281379 and 2009/0281557, which are also commonly owned, describe stents and other tissue anchors of the type that can be deployed by the apparatus of the present invention. U.S. Published Application No. 2003/069533 describes an endoscopic transduodenal biliary drainage system which is introduced through a penetration, made by a trans-orally advanced catheter having a needle which is advanced from the duodenum into the gallbladder. US 6,620,122 describes a system for placing a self-expanding stent from the stomach into a pseudocyst using a needle and an endoscope. US 2005/0228413, commonly assigned with the present application, describes a tissue-penetrating device for endoscopy or endosonography-guided (ultrasonic) procedures where an anchor may be placed to form an anastomosis between body lumens, including the intestine, stomach, and gallbladder. See also US 5,458,131; US 5,495,851; US 5,944,738; US 6,007,522;US 6,231,587; US 6,655,386; US 7,273,451; US 7,309,341; US 2004/A2$122;U52004/0249985; US 200710123917; WO 2006/062996;EP 1314404 Kahaleh et al. (2006) Gastrointestinal Endoscopy 64:52-59; and Kwan et al. (2007) Gastrointestinal Endoscopy 66:582-586. Shaped balloons having differently sized segments and segments with staged opening pressures are described in U.S. Patent Nos. 6,835,189; 6,488,653; 6,290,485; 6,022,3 59 ; 5, 843, 1I6; 5,620,457 ; 4,99 0,139 ; and 3,97 0,090. US 2010/0268029 discloses a guidewire having a guidewire body with a distal end and a proximal end. A balloon or other tissue anchor is disposed at or near the distal end of the guidewire, and the guidewire may be used to draw two layers of tissue into apposition by placing the guidewire through a tissue penetration, deploying the tissue anchor, and drawing proximally on the guidewire body. EP 0829238 discloses a device for incision and dilation of stenotic segments within the vascular system of a patent, which includes a rigid ellipsoidal dilation probe mounted at the distal end of a catheter.

### SUMMARY OF THE DISCLOSURE

The present invention provides a catheter as defined in the appended claims.

Described herein are methods and apparatus for advancing a dilator distally through apposed luminal walls of the adjacent first and second body lumens. With respect to some methods, a needle is distally advanced through the luminal walls to create an initial passage therethrough. An anchor disposed on the needle itself is then deployed on a distal side of the distal-most luminal wall, and the deployed anchor is drawn proximally against the distal most luminal wall by drawing or pulling the needle in the proximal direction. In this way, the luminal walls may be held together during subsequent steps of the procedure. In the next step, a dilator is usually advanced over the needle to enlarge the penetration where a distal tapered region of the dilator is advanced past the deployed anchor in order to assure that the dilator is able to pass completely through the penetration and achieve full dilation.

In a first specific example of a method, deploying the anchor comprises radially expanding a plurality of wings which are disposed on or formed as part of the needle structure. For example, a tubular wall of the needle may be axially split in order to form two, three, four, or more axial segments which can be radially expanded by axially foreshortened tubular wall, e.g., either by pulling a distal portion of the needle wall proximally or by advancing a proximal portion of the needle wall distally. Such structures are commonly referred to in the medical device arts as "malecot" structures and are similar in design to common molly bolts.

In a further specific example of some methods, the distal tip of the dilator will be adapted to permit it to physically pass at least a portion of the deployed anchor structure. For example, when the anchor structure comprises a plurality of radially expanding wings as described above, the dilator tip may be formed to have a plurality of axial slots arranged to receive the deployed wings and allow the dilator tip to pass by said wings. The distal end of the catheter may also have a plurality of axial slots which are in alignment with the slots on the dilator tip and are arranged to receive the deployed wings and to allow both the dilator tip and the catheter to pass at least a portion of said wings. Depending on the specific structure of the anchor, however, a variety of other designs could be implemented to allow such bypass.

In a further specific example of some methods, the dilator will include a blade having a cutting edge disposed in a distal direction so that advancing the dilator through the penetration causes the blade to cut a peripheral portion of the luminal walls which surround the penetration. Typically, the blade will be advanceable or reciprocatable with respect to the dilator so that the blade may remain retracted in a safe mode while the catheter is being introduced to the target site within a first body lumen. Only when the dilator is ready to be advanced through the wall is the blade then itself advanced to expose the blade to the tissue as the dilator is passed therethrough. In a specific example, a single blade, is oriented to be advanced from the dilator and will travel through a channel or track formed in the needle so that the blade remains closely adjacent to the needle as it is advanced.

It has been found by the inventors herein that passing the dilator, even with the blade extended, can be very difficult in many tissues. Applicants herein have found that there is a very thin but strong membrane disposed between many tissues of interest, such as between the mucosa and muscle layer of the stomach and intestine or adjacent the lining of the gastrointestinal track on the peritonel side. While the membrane is very thin (being about one quarter to one half the thickness of paper), it is very strong and tends to stretch around the advancing dilator tip and to get caught on any edge or space between any components of the dilator tip. By way of example, the membrane can get caught between the dilator and/or the blade as the dilator and blade are advanced over the needle. By advancing the blade through a channel or track in the needle rather than on an outer surface of the needle, the risk of being caught in this tissue layer is greatly reduced. The risk of catching this membrane is also reduced by making sure that there are no edges or spaces anywhere along the needle, dilator or blade.

Also described herein is a catheter for forming and dilating a passage through apposed luminal walls comprises a catheter body having a proximal end, a distal end, and a central passage therethrough. The catheter further includes a dilator tip which is coupled to the distal end of the catheter body, typically being fixedly attached thereto. A needle having a tissue-penetrating tip, typically a sharpened or a honed tip, but alternatively being a radiofrequency or other energy-based penetrating tip, is reciprocatably mounted in the central passage of the catheter so that the distal tip of the needle can be advanced beyond the dilator tip of the catheter body to penetrate the luminal walls to create an initial tissue penetration therethrough. A deployable anchor structure is disposed on the needle and is adapted to be expanded and drawn proximally by the needle to bring luminal walls that may be separated into apposition and hold the luminal walls together as the dilator tip is advanced therethrough. A blade is mounted to be distally advanced from the dilator tip to cut the tissue passage as the dilator tip is advanced through the tissue passage.

According to the invention, the anchor structure will typically comprise a plurality of radially expandable wings, generally as described above with respect to the methods. Similarly the dilator tip may have a plurality of axially slots with or without a plurality of aligned axial slots in the distal end of the catheter, arranged to receive the radially expanded wings as the dilator tip is advanced over the needle, also as generally described above with respect to the methods. The expandable wings will preferably be aligned to the receiving slots in the dilator tip and, if used, the receiving slots in the distal catheter end. Alignment of the three expandable wings to the three receiving slots in the dilator tip and the catheter end, allow the dilator and catheter to be advanced past at least a portion of the radially expanded wings, also as described generally above with respect to the method. Finally, the apparatus will often carry a radially expandable stent on a distal region of the catheter body so that the stent can be deployed within the dilated passage formed through the apposed luminal walls.

In a further specific embodiment of the apparatus, certain structural changes are provided to reduce the likelihood that tissue will be inadvertently captured in the small space under the blade as the dilator is advanced through tissue and to reduce the profile of the catheter body and improve the flexibility thereof. In particular, the cutting blade may be received in an extended channel, which is proximal to the dilator nose cone and along the longitudinal axis of the needle. The blade may then be advanced through a channel in the needle, a feature that will reduce the likelihood of tissue being inadvertently captured in gaps between the blade and the supporting structure. In some embodiments, the blade can be coupled to a retractable sheath which is used to deploy the stent. By coupling the blade to the sheath rather than to a separate rod or other deployment element within the catheter, the flexibility of the catheter can be improved and the diameter or profile reduced. In other embodiments, the blade may be automatically deployed by the distally advancing needle, without any separate actuator coupling it to the proximal handle of the instrument. In some embodiments, the blade may be moved radially outward as it is also deployed in a distal direction from a slot in the dilator nose-cone.

### BRIEF DESCRIPTION OF THE DRAWINGS

Fig. 1 is a perspective view of a dilation catheter constructed in accordance with the principles of the present disclosure.
Figs. 2-6 illustrate the distal end of the catheter of Fig. 1 showing the relative deployment movements of a dilator tip, a tissue-penetrating needle, a deployable anchor, and a cutting blade on said catheter.
Figs. 7 and 8 illustrate two general embodiments of a handle useful for deploying the various components of the catheter of Fig. 1.
Figs. 9A-9E illustrate use of the catheter of Fig. 1 for penetrating and dilating apposed luminal walls of adjacent first and second body lumens.
Fig. 10 is a perspective view of a second embodiment of a dilation catheter constructed in accordance with the principles of the present disclosure.
Fig. 10A is a detailed view of the distal end of the catheter in Fig. 1 showing the gap between the nose cone and the sheath.
Fig. 11 shows setting of the trocar depth collar which is performed prior to advancement of the trocar needle from the supporting catheter shaft.
Figs. 12, 12A and 12B illustrate advancement of the needle from the supporting catheter body.
Figs. 13, 13A and 13B illustrate deployment of the anchor structure in the target body lumen.
Figs. 14, 14A and 14B illustrate deployment of the cutter blade by sheath advancement.
Figs. 15, 15A and 15B illustrate advancement of the sheath and deployed cutter blade through the tissue with the anchor being received in the anchor channels in the nose cone and the distal sheath component.
Figs. 16, 16A and 16B illustrate deployment of the distal flange of the stent by partial retraction of the outer sheath.
Figs. 17, 17A and 17B illustrate deployment of the proximal flange of the stent by complete retraction of the outer sheath.
Figs. 18-21 illustrate details of the distal end of another exemplary dilator catheter embodiment.
Figs. 22-23 illustrate details of the proximal end of the dilator catheter shown in Figs. 18-21.
Figs. 24-26 illustrate another anchor embodiment.

### DETAILED DESCRIPTION

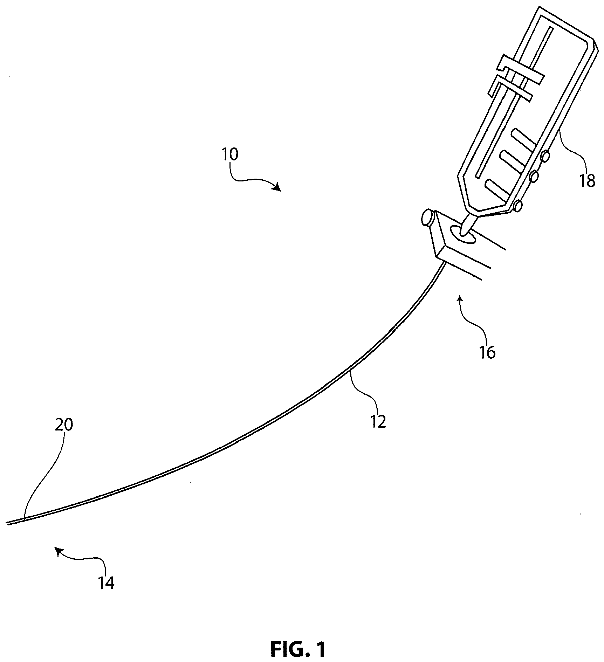

Referring to Fig. 1, a dilation catheter 10 constructed in accordance with the principles of the present disclosure, comprises a catheter body 12 having a distal end 14 and a proximal end 16. A handle 18 is attached at the proximal end 16 of the catheter body and a penetration and dilation assembly 20 is located at the distal end of the catheter body 12.

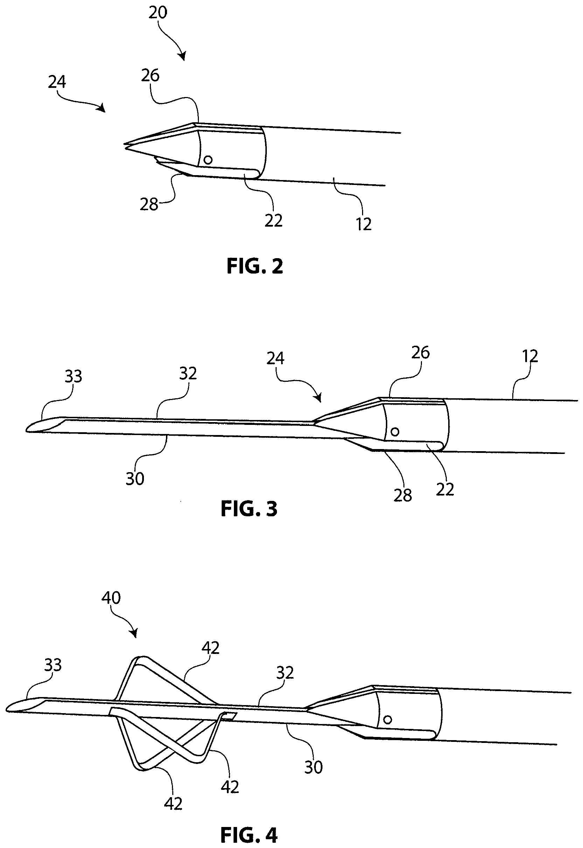

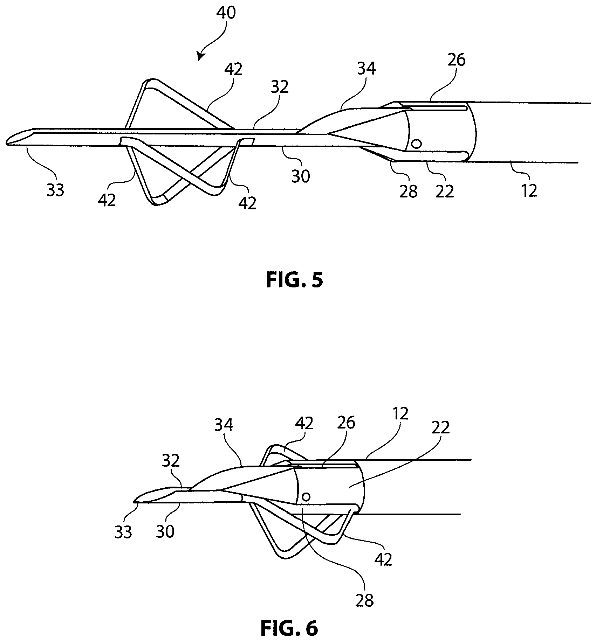

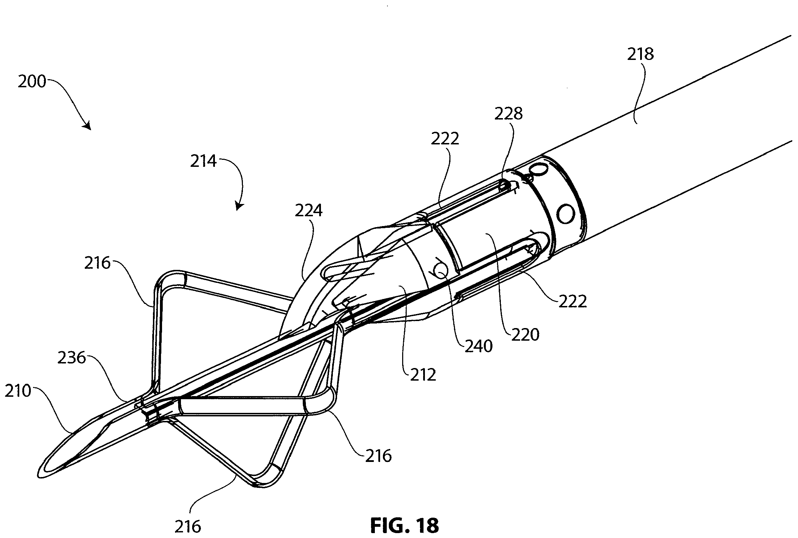

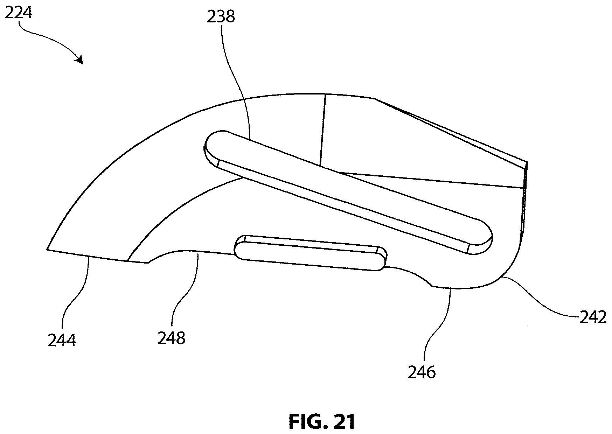

Referring now to Figs. 2-6, construction and deployment of an exemplary penetration and dilation mechanism 20 will be described. A dilator tip 22 has a tapered distal end 24 which has a blade slot 26 and three anchor-accommodating slots 28 formed therein. It is possible, if desired, to exclude a blade if not required in a particular tissue type or thickness. In such embodiments, the penetration and dilation mechanism 20 can dilate and advance through the tissue layers without the blade 34. In some embodiments having a blade, the blade may slide forward in manner that remains at its initial height relative and parallel to the axial slot 32, as in Figs. 5 and 6. In a second configuration, the blade can be elevated from its initial height as it moves distally forward by placing an angled guide slot from proximal to distal ends as in Figs. 18-21. The blade may also have a side bar which controls blade depth, secures the lower surface of blade in an axial slot and prevents unwanted movement such as side to side and/or blade tipping movement. Friction is often a problem in coaxial catheter devices. To reduce friction as the blade is advanced distally or retracted proximally a cut out located on the lower surface of the blade can optionally be used. The blade may include other features that reduce the friction of the blade or act as actuators as the blade is moved in the distal or proximal direction, as shown in Fig 21. A central passage through the dilator tip 22 and also through the remaining length of the catheter body 12 reciprocatably receives a tissue-penetrating needle 30, as best seen in Figs. 3-6. A tissue-penetrating needle has a beveled or honed distal end 33 and an axial slot 32 formed along at least its distal length. The slot 32 reciprocatably receives a cutting blade 34, as best seen in Figs. 5 and 6. The base of the cutting blade 34 travels through the axial slot 32 and into the lumen of the tissue-penetrating needle 30 where, if desired, the base of the blade is reciprocatably held in place. As is apparent from those figures, the axial slot 32 is aligned with the blade slot 26 on the dilator tip 22 so that the blade may be distally advanced from the blade slot 26 to travel through the axial slot 32 which reduces or eliminates the presence of gaps between the blade and the rest of the structure which may catch tissue during penetration as described earlier in this application.

An anchor mechanism 40 comprising three radially expandable wings 42 is attached over the exterior surface of the needle 30. The wings 42 lie flush with the exterior surface of the needle 30, as shown in Fig. 3, and are deployed radially outwardly by axially foreshortening the wings so that they move freely outward as shown in Figs. 4-6. Such axial shortening can be achieved in numerous ways well known in the medical device arts. For example, the wings can be formed in the walls of an outer tube (not specifically illustrated) by, for example, cutting slits in the outer tube. The outer tube may then be foreshortened by pushing on the proximal end and/or pulling proximally on the distal end in order to achieve the desired deformation of the wings. The wings can have various lengths and widths which produce a variety of wing configurations and strengths. Each wing may be constructed with multiple widths, may be etched, and/or may be made of preformed memory metal or by other means which biases the wings to a predetermined expanded configuration, as will be subsequently described in more detail.

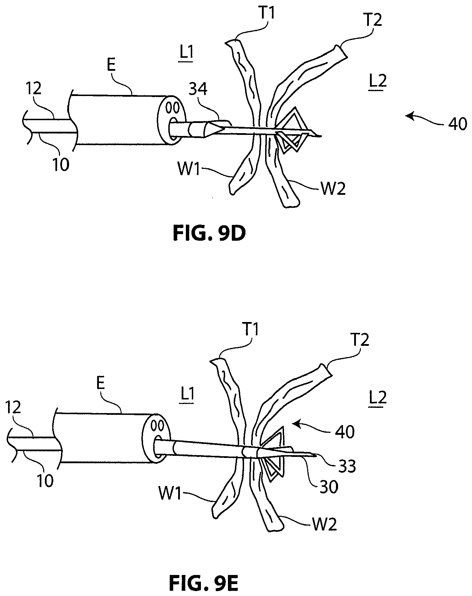

Once the needle 30 has been extended, the anchor mechanism 40 deployed, and blade 34 axially advanced, as shown in Fig. 5, the subassemblies of catheter body 12 and the penetration and dilation mechanism 20, including the dilator tip 22, and cutting blade 34, may be axially advanced over the needle 30 with the wings 42 being received in the anchor slots 28, as shown in Fig. 6. The ability to advance the penetration and dilation mechanism 20 with deployed blade 34 past the wings 42 of the anchor mechanism 40 is advantageous since it allows penetration and dilation mechanism 20 including the dilator tip and the blade to pass fully through the tissue while that tissue remains compressed or held together and in place by proximal tension applied through the anchor mechanism. This advantage can be best seen in Fig. 9E discussed below.

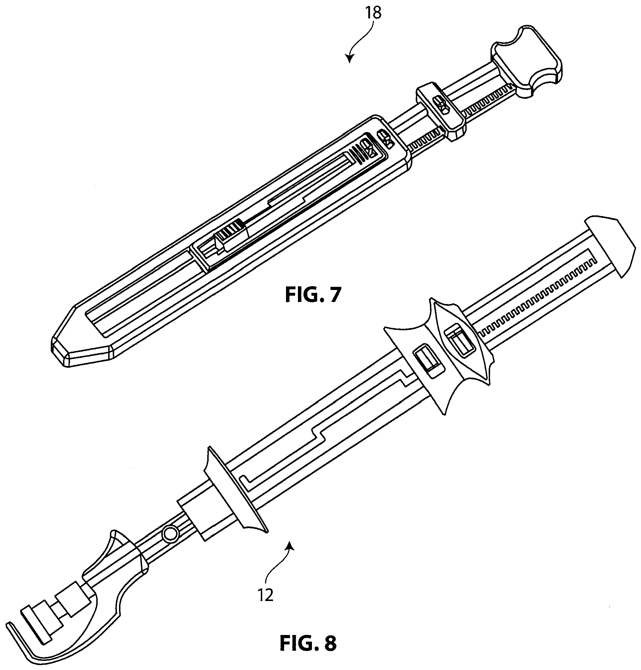

Two exemplary handle mechanisms 18 are illustrated in Figs. 7 and 8, respectively.

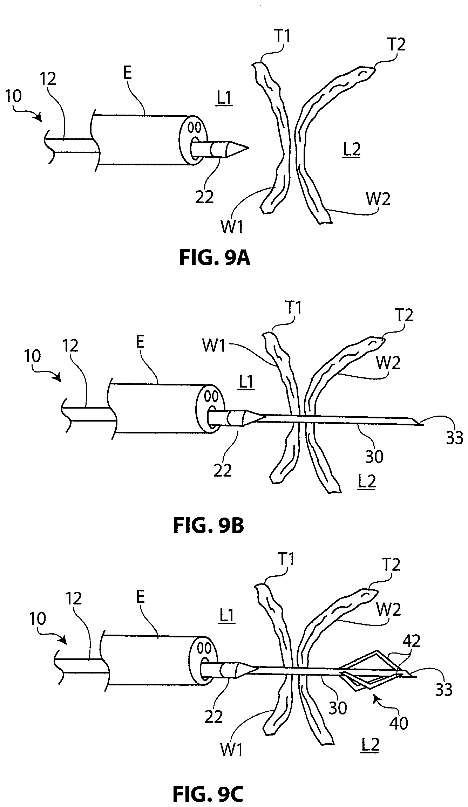

Referring now to Figs. 9A - 9E, use of the exemplary catheter 10 for penetrating and dilating the passage through adjacent tissue layers T1 and T2 and body lumens L1 and L2 will be described. Body lumen L1 has a front or anterior wall W1 while body lumen L2 has a back or posterior wall W2. The catheter body 12 is advanced through a working lumen of endoscope E so that the dilator tip 22 lies adjacent to and directed toward a target location on the front wall W1 of body lumen L1 as shown in Fig. 9A. Once in this position, the needle 30 can be advanced from the dilator tip 22 through the wall W1 and tissue layers T1 and T2, as shown in Fig. 9B. After the tip 33 of the needle 30 has been advanced well past the back wall W2 of tissue layer T2 in lumen L2, the wings 42 of the anchor mechanism 40 can be radially expanded, as shown in Fig. 9C. The needle 30 can then be drawn proximally to pull the anchor mechanism 40 against the rear wall W2 of tissue layer T2, as shown in Fig. 9D, to compress the layers T1 and T2 together. Blade 34 may then be advanced from the dilator tip 22, also shown in Fig. 9D. The catheter body 12 can then be advanced distally to push the blade 34 and tapered end of dilator tip 22 through the luminal walls T1 and T2, as shown in Fig. 9E. The penetrating mechanism 40 of catheter body 12 will reach the fully advanced configuration of the needle and dilation tip 22 as illustrated in Fig. 6, thus assuring that the tissue layers T1 and T2 are fully penetrated and dilated.

Optionally, as described in more detail for example in co-pending U.S. application number 12/772,762 filed May 3, 2010, and in U.S. application number 12/790,553 filed May 28, 2010, a stent carried at the distal end of the catheter body 12 immediately proximal to the dilator tip 22 can then be released to span the penetration that has just been formed and dilated. For example, the stent can be self-expanding and released by proximally retracting a sheath which covers the stent and retains it in its collapsed (low profile) configuration until it is desired to deploy the stent.

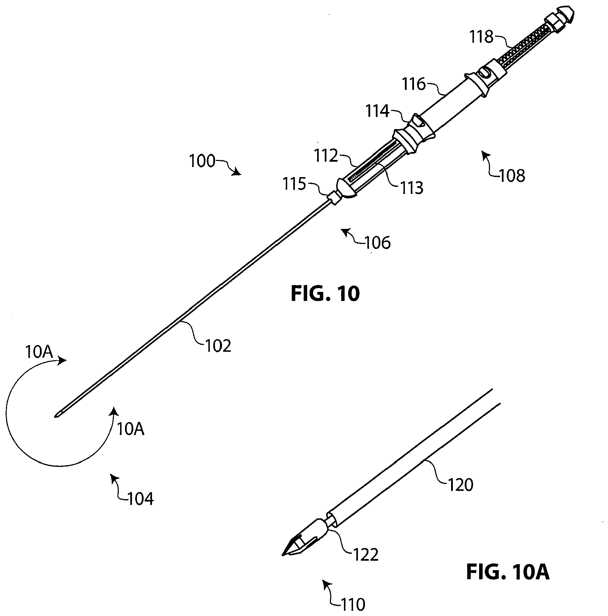

Referring to Figs. 10, and 10A, dilation catheter 100 constructed in accordance with the principles of the present disclosure comprises a catheter body 102 having a distal end 104 and a proximal end 106, a handle assembly 108, and a penetration and dilation assembly 110.

The handle assembly 108 includes a nose 112 with luer fitting 115 at the distal end which allows the user to secure the catheter to an endoscope after the catheter body or shaft 102 has been inserted into the endoscope's working channel. The nose 112 will also include a depth indicator or scale 113 which provides a visual indication of the depth of catheter advancement from the distal end of the endoscope. A trocar or needle handle 118 controls advancement and retraction of the trocar/needle, as described in more detail below. A catheter handle 116 controls movement of the catheter shaft relative to the nose 112 so that the catheter shaft may be advanced from the endoscope. A sheath control hub 114 is coupled to an outer sheath 120 of the catheter body 102 which radially constrains the stent (described below) prior to deployment. In some embodiments, the sheath control hub 114 may also be used to advance and retract a cutter blade 130 by advancing the sheath over a gap 122 between the sheath and the penetration and dilation assembly 110, as described in more detail below.

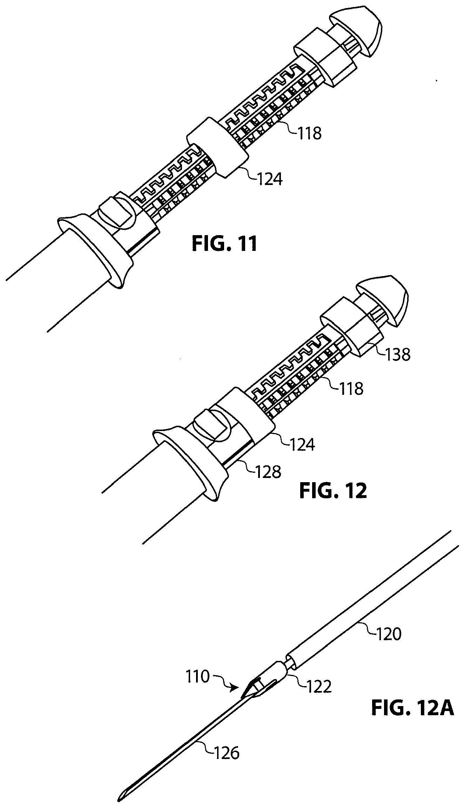

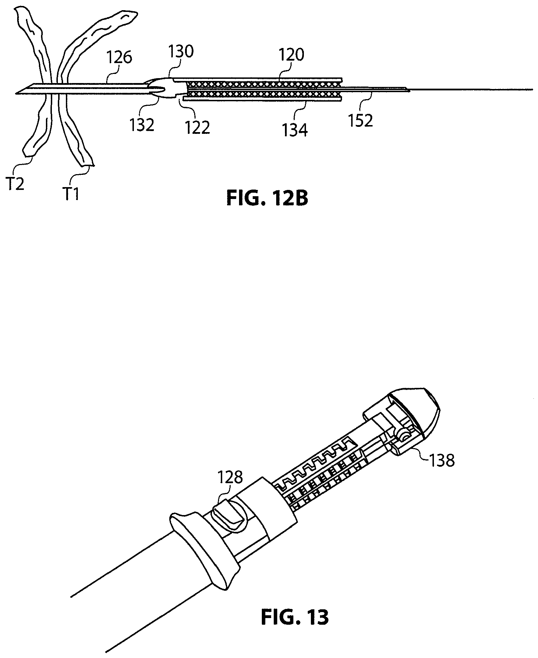

Referring now to Fig. 11, after the catheter body 102 has been advanced through an endoscope (not shown) a depth control collar 124 is positioned on the trocar/needle handle 118 according to a scale thereon and a desired penetration depth. he trocar handle is then thrust forward (at a distal direction) to advance a trocar/needle 126 from the penetration and dilation assembly 110, as illustrated in Figs. 12, 12A, and 12B. The trocar handle 118 is advanced until the depth control collar 124 reaches a proximal surface of a trocar handle lock 128 which extends upward from the proximal end of the catheter handle 116. In this way, the trocar/needle 126 will be advanced by a predetermined distance, as shown in Figs. 12A and 12B. As shown particularly in Fig. 12B, the needle/trocar 126 will be advanced through tissue layers T1 and T2. At this point, blade 130 remains within a nose cone 132, and self expanding stent 134 remains radially constricted within the outer sheath 120.

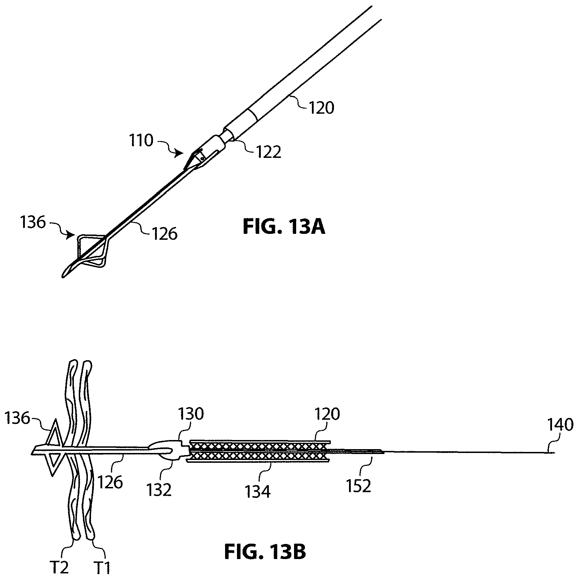

Referring now to Figs. 13, 13A, and 13B, an anchor 136 is deployed on the distal side of tissue layer T2 by drawing proximally on an anchor actuator 138 to retract a pull wire 140 which runs through a central lumen in the trocar/needle 126. The anchor will typically comprise a malecott structure which is formed by providing axial slits in the body of the trocar/needle 126. Such structures are described, for example, in commonly owned, co-pending patent application nos. 12/757,408; 12/757,421; and 12/772,762.

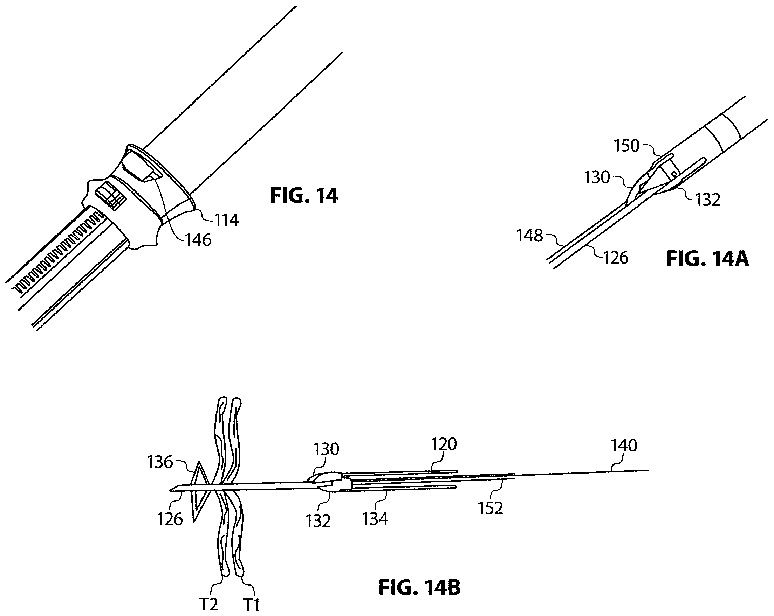

Referring now to Figs. 14, 14A, and 14B, the blade 130 may be advanced by unlocking the sheath control hub lock 146 and distally advancing the sheath control hub which is coupled to the outer sheath 120. In this way, the outer sheath 120 advances distally relative to the inner shaft member 152 so that the sheath closes the gap 122 (Fig. 10A) and advances relative to the nose cone 132. The sheath 120 is directly coupled to the blade 130, thus causing the blade to advance from the blade channel 150 and nose cone 132. Additionally, in order to reduce the gaps between the blade 130 and the trocar/needle 126, the blade advances in a channel 148 formed in the trocar/needle. Thus, the risk of the blade unintentionally capturing tissue, either tearing the tissue or preventing advancement of the trocar, is greatly reduced.

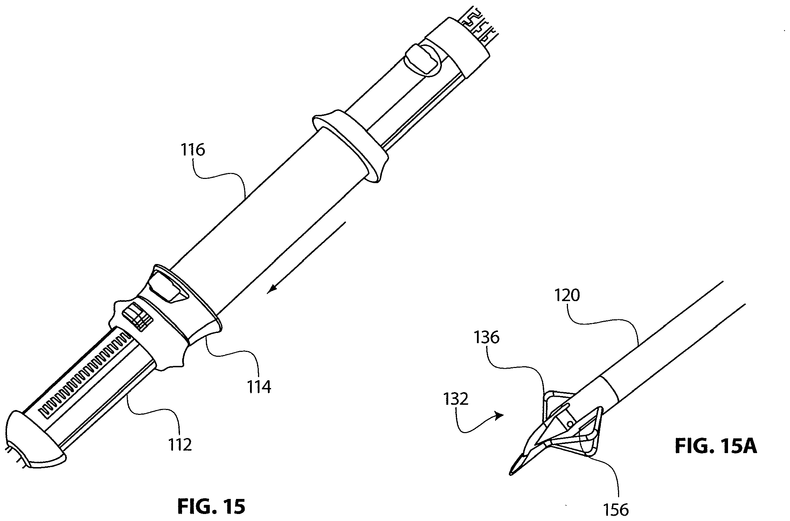

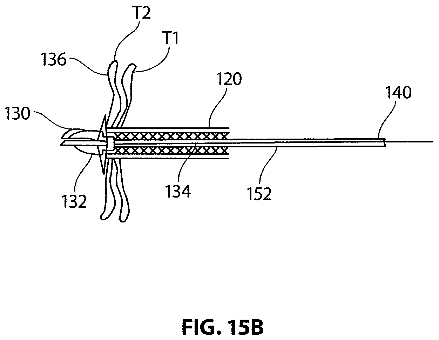

Referring now to Figs. 15, 15A and 15B, the catheter body or sheath 102 (Fig. 10) is advanced so that the tapered, dilating end of the nose cone 132 advances over the needle/trocar 126 and dilates the small hole in tissue layers T1 and T2. Tension on the anchor 136 is maintained by simultaneous proximal traction on the needle 126 and the pull wire 140.

The blade 130 is advanced so that channels 156 in the nose cone 132 receive the elements of the anchor 136, as best seen in Fig. 15A. This allows the blade 130 and tapered dilating tip of the nose cone 132 to extend beyond the anchors 136 which engage the posterior surface of the proximal-most tissue layer T2, as best seen in Fig. 15B. After the nose cone 132 has been fully advanced over the anchor 136, as shown in Fig. 15A, the catheter handle is locked relative to the inner shaft, and the stent 134 is ready to be deployed.

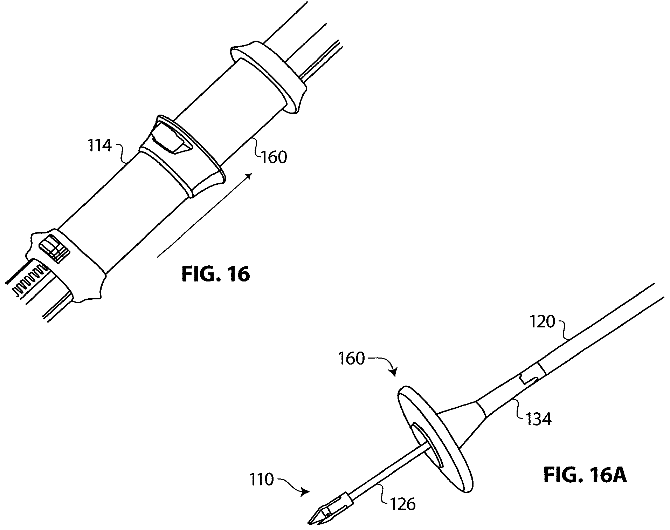

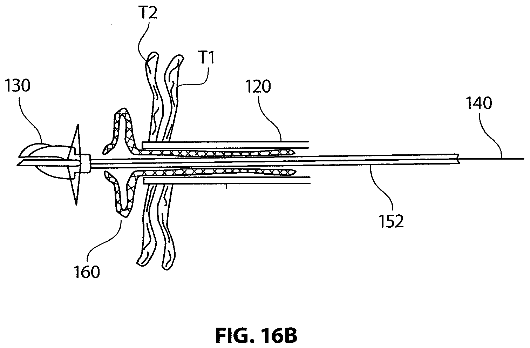

As illustrated in Figs. 16, 16A, and 16B, the sheath control hub lock 146 is disengaged, allowing the sheath control hub 114 to be drawn proximally along the catheter handle 116 to retract the sheath 120 from over the distal end of the stent 134. As the outer sheath 120 is retracted, a distal flange 160 on the stent 134 will deploy from the proximal or posterior side of the second tissue layer T2, as shown in Fig. 16B. The flange 160 can then be pulled against the proximal tissue layer T2 to hold the layers T1 and T2 together.

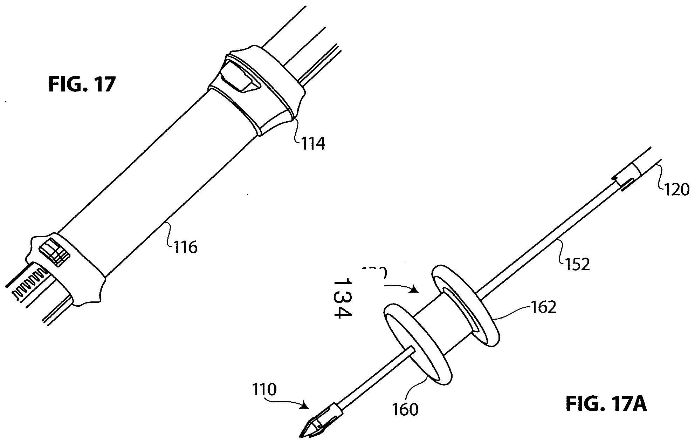

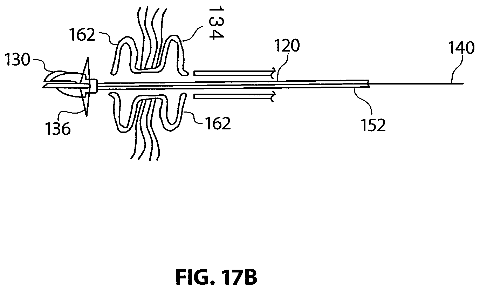

As shown in Figs. 17, 17A, and 17B, deployment of a proximal flange 162 on stent 134 is accomplished by fully retracting the outer sheath 120 over the inner shaft 152. To do so, the sheath control hub 114 is completely drawn proximally over the catheter handle 116. After the stent 134 is deployed, the catheter assembly may be withdrawn through the endoscope after the anchor has been collapsed and the needle has been retracted into the catheter, leaving the fully deployed stent in place in the tissue layers T1 and T2.

Referring to Figs. 18-23, another exemplary dilation catheter 200 constructed in accordance with the principles of the present disclosure is shown. Dilation catheter 200 is constructed and functions in a manner similar to that of previously described dilation catheter 100, but with some modifications that will be subsequently described in detail.

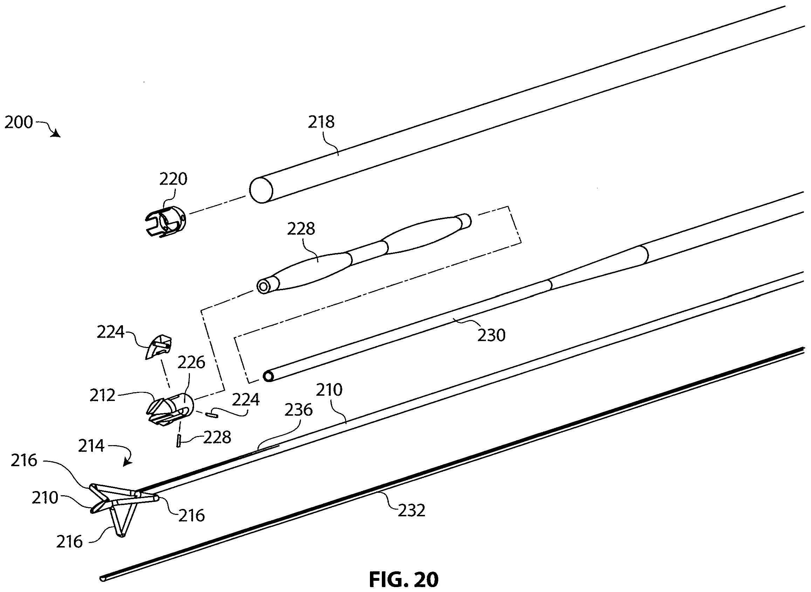

Fig. 18 is an enlarged perspective view showing the distal end of dilation catheter 200. As with previously described catheter 100, catheter 200 includes needle 210 reciprocatingly received within dilator tip 212. Anchor structure 214 is formed in part by needle 210 and includes three wings 216, which are shown in their radially deployed positions in Fig. 18. The distal end of catheter 200 also includes the distal tip of outer sheath 218. Unlike previously described catheter 100, the distal tip of outer sheath 218 of catheter 200 is provided with a slotted crown 220. Slotted crown 220 includes four slots 222, similar to the four slots provided in dilator tip 212, for receiving the deployed wings 216 of anchor structure 214 and blade 224. As best seen by comparing Figs. 18 and 20, dilator tip 212 has a reduced diameter portion 226 on its proximal end that is received within the inner bore of slotted crown 220 when outer sheath 218 is fully extended distally against dilator tip 212. A vertically extending pin 228 protrudes from the top surface of dilator tip 212 and is slidably received within the top slot of crown to ensure that slots 222 are properly aligned rotationally for receiving wings 216 and blade 224. As described below, the vertically extending pin 228 also protrudes from the internal diameter of the dilator tip 212 and is slidably received within the top longitudinal slot of the needle 210 to ensure that the deployed wings 216 of anchor structure 214 are properly aligned rotationally for the slots in the dilator tip 212.

Referring to Fig. 20, dilator 200 is provided with an expandable stent 228, similar in construction and operation to the stent described in reference to previous embodiments. Before deployment, stent 228 resides on pusher catheter 230 and is held in its radially compressed and axially expanded state by outer sheath 218. The distal end of stent 228 may be butted up against the proximal end of dilator tip 212, which is rigidly affixed to the distal end of pusher catheter 230. In some embodiments, the distal end of stent 228 may extend into an annular recess within the proximal end of dilator tip 212. Once the distal end of outer sheath 218 is in the desired location, stent 228 is deployed one flange at a time by retracting outer sheath 218 in a proximal direction relative to pusher catheter 230 and stent 228.

The arrangement of slotted crown 220 described above advantageously allows the distal tip of outer sheath 218 to be fully advanced through both tissue layers being penetrated while proximal tension on the tissue layers is being applied by anchor structure 214. This occurs because slots 222 in crown 220 allow the majority of crown 220 to advance past at least a portion of anchor structure 214, namely, past the proximally facing legs of wings 216 which are applying force in a proximal direction on the distal most tissue surface. Once the majority of crown 220 is advanced fully through both tissue layers, wings 216 may be collapsed to their retracted state and dilator tip 220 and outer sheath 218 may be further advanced distally with respect to needle 210 and the tissue without the need for anchor structure 214 to apply a proximal force against the tissue. Alternatively, wings 216 may remain deployed with the entire distal tip of catheter 200 (i.e. needle 210, wings 216, dilator tip 212, slotted crown 22 and outer sheath 218) further advanced distally with respect to the tissue. Further advancement of the catheter tip through the tissue ensures that when stent 228 is deployed, its distal flange is distally beyond the distal-most tissue and will not pull back proximally through the passage that has been created through the tissue walls. In some embodiments of the method disclosed herein, slotted crown 220 is distally advanced through the tissue about 1 cm more after slots 222 of crown 220 have bottomed out on deployed wings 216.

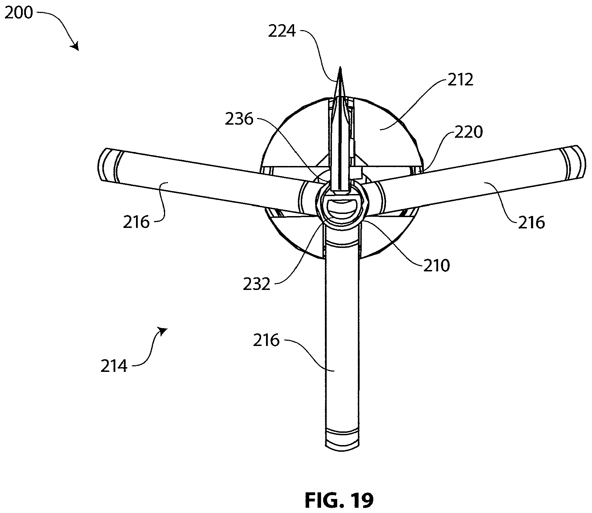

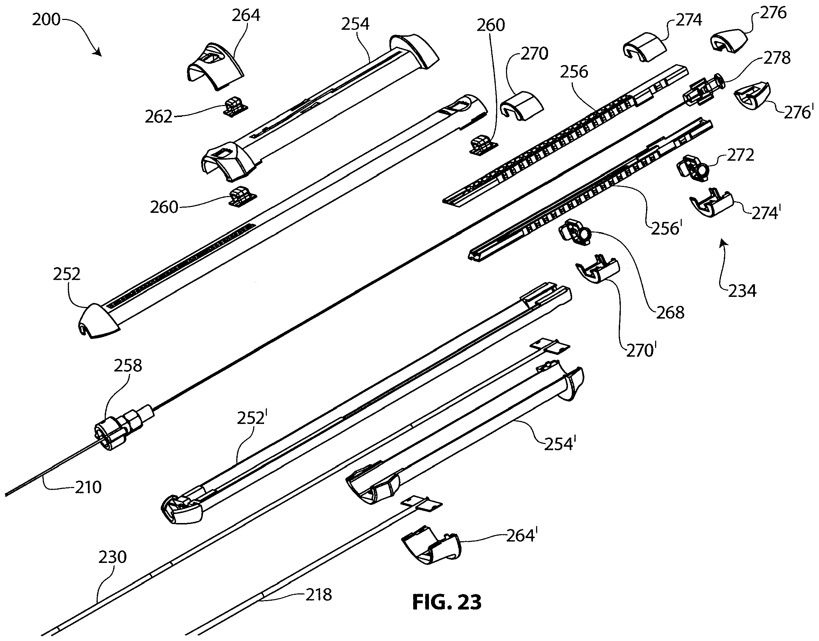

Referring to Figs. 19 and 20, a pull tube 232 having a D-shaped cross-section may be provided through the lumen in needle 210. At its distal end, pull tube 232 may be welded to the distal end of needle 210, distally of anchor wings 216. At its proximal end, pull tube 232 may be coupled to an anchor actuator 234 (shown in Fig. 23). Similar to previous embodiments, anchor wings 216 are outwardly deployed by retracting pull tube 232 proximally with respect to needle 210 using anchor actuator 234. In this embodiment, a slot 236 is provided through the top wall along the distal 8 cm of needle 210. A bottom portion of pin 228 (as best seen in Fig. 20) residing in dilator tip 212 is slidably received in needle slot 236 to keep dilator tip 212 and slotted crown 220 rotationally aligned with wings 216 on needle 210. D-shaped pull tube 232 and needle slot 236 cooperate to provide a support to slidably receive the bottom of blade 224, as best seen in the end view of Fig. 19. D-shaped pull tube 232 also provides a conduit through needle 210 that may be used for aspiration and/or irrigation, as is subsequently described in more detail. In other embodiments, the distal end of the pull tube may take the shape of a smaller diameter tube that is affixed to the bottom of the needle lumen near its distal end.

Referring to Fig. 21, blade 224 may be provided with a slot 238 for movably coupling the blade to dilator tip 212. As depicted in Fig. 20, blade 224 is slidably received within the top slot of dilator tip 212 and is movably coupled thereto with pin 240, which passes through blade slot 238 and both sides of dilator tip 212. When needle 210 is fully retracted in catheter 200, its distal tip is located proximal to dilator tip 212 and blade 224. In this configuration, blade 224 is able to drop down within needle slot 236 until the bottom of blade 224 contacts the bottom interior wall of needle 210. As can be appreciated by viewing the end view of Fig. 19, when blade is in this lowered position (not shown), its cutting edge is safely recessed within the outer circumference of dilator tip 212. As needle 210 is extended from the distal end of catheter 200, the beveled distal tip of needle 210 (shown in Fig. 18) contacts the proximal, rounded bottom corner of blade 224 (shown in Fig. 21) to actuate the blade. Angled slot 238 allows blade 224 to move distally and radially outwardly as it is being actuated by needle 210. Lower contact surfaces 244 and 246 allow blade 224 to slide along the top surface of D-shaped pull tube 232 as needle 210 is further extended, while recessed area 248 reduces friction between blade 224 and pull tube 232. A side bar 250 may extend from one or both sides of blade 224 and engage with a mating surface within dilator tip 212 to limit outward movement and pivoting of blade 224. A spring or other biasing member (not shown) may be provided between blade 224 and vertical pin 228 or other feature to assist with the downward and proximal retraction of blade 224 when needle 210 is retracted. As previously described, having the bottom of blade 224 recessed within needle slot 236 during operation inhibits membranes and other tissue from getting caught between blade 224 and needle 210.

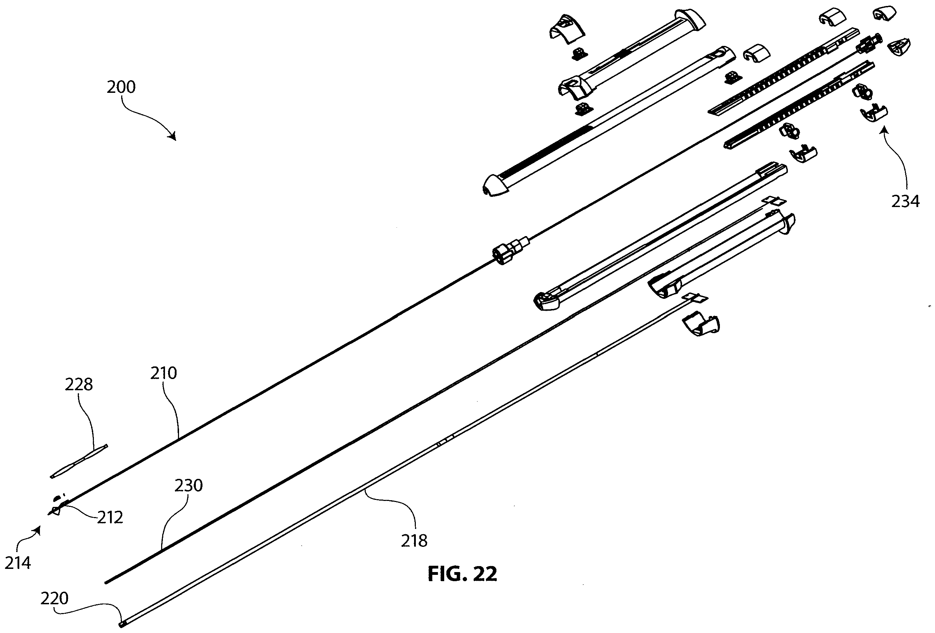

Referring to Figs. 22 and 23, components of the proximal end of dilation catheter 200 are shown. In this embodiment, catheter 200 includes handle nose 252, 252', pusher top 254, 254', needle handle 256, 256', swivel luer fitting 258, catheter lock 260, outer sheath lock 262 and actuator 264, 264', needle lock 266, needle depth button 268 and handle 270, 270', pull tube button 272 and handle 274, 274', back handle 276, 276', and luer fitting 278. Luer fitting 278 may be configured for attaching a syringe or tubing (not shown) to the proximal end of catheter 200. In this embodiment, luer fitting 278 is in sealed fluid communication with the lumen of D-shaped pull tube 232 which extends to the distal end of needle 210. With this arrangement, fluid may be aspirated through catheter 200 from adjacent the distal tip, such as to confirm that the distal tip is in the desired location by inspecting the aspirated fluid, to clear the operating field, and/or to sample patient fluid for testing. Fluid(s) may also be supplied to the operating field through catheter 200, such as for supplying irrigation fluid or a contrast agent for imaging.

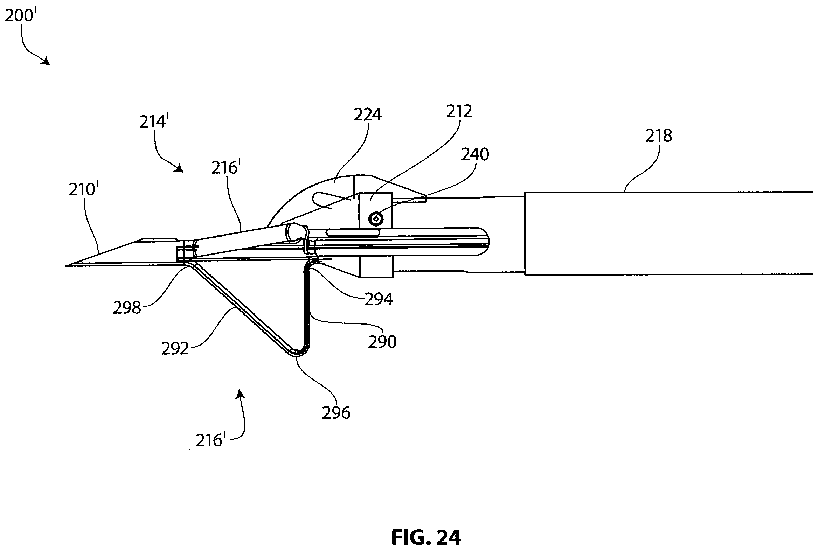

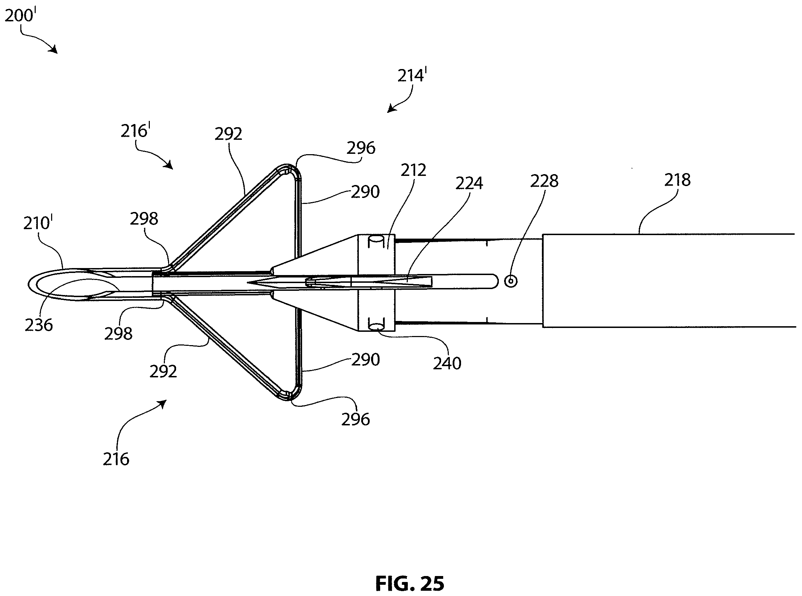

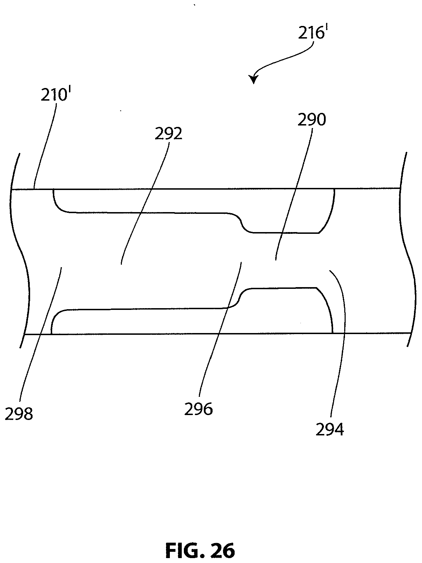

Referring to Figs. 24-26, an alternative anchor structure 214' is shown. This exemplary structure is shown in a deployed configuration in Figs. 24 and 25 and a non-deployed configuration in Fig. 26. Anchor structure 214' includes three outwardly deployable wings 216', similar to those previously described. As shown in Figs. 24 and 25, wings 216' are configured with proximal legs 290 that are substantially orthogonal to the main axis of catheter 200'. This arrangement provides more contact area with a tissue surface being anchored against. Additionally, since proximal legs 290 are configure to lie generally flat against the tissue rather than being outwardly angled as in previous embodiments, there is less of a tendency for anchor structure 214' to dilate the passage through the tissue and slip back through the tissue walls. In this embodiment, the distal legs 292 of wings 216' are configured to have an angle of about 45 degrees with the main axis of the catheter.

Fig. 26 illustrates the construction features of the exemplary anchor structure 214'. Proximal leg 290 may be made thinner and shorter than distal leg 292. This configuration allows wing 216' to bend at points 294, 296 and 298 when deployed. The pattern depicted may be fabricated with a laser cutter, electro-discharge machining or other suitable methods. In some embodiments, the length of proximal legs 290 is about 0,38 cm (0.15 inches) and the length of distal legs 292 is about 1,02 cm (0.40 inches).

Although the foregoing invention has been described in some detail by way of illustration and example, for purposes of clarity of understanding, it will be obvious that various alternatives, modifications and equivalents may be used and the above description should not be taken as limiting in scope of the invention which is defined by the appended claims.

## Claims

1. A catheter for creating a passage through apposed luminal walls and stent delivery, the catheter comprising;
a catheter body (102) having a proximal end (106), a distal end (104), and a central passage therethrough;
a dilator tip coupled to the distal end of the catheter body (102);
a needle (126) having a penetrating distal tip, said needle (126) being reciprocatably mounted in the central passage so that said distal tip can be advanced beyond the dilator tip of the catheter body (102) to penetrate the apposed luminal walls;
an anchor structure deployable from the needle (126) and comprising a plurality of radially expandable wings, said anchor structure adapted to be expanded and drawn proximally by the needle (126) against the apposed the luminal walls ;
a blade (130) extendable from the dilator tip to cut the passage as the dilator tip is advanced through the passage; and
a radially expandable stent (134) carried on a distal region of the catheter body (102).

2. A catheter as in claim 1, wherein the dilator tip has a plurality of slots arranged to receive the radially expanded wings as the dilator tip is advanced over the needle (126).

3. A catheter as in claim 2, wherein the expandable wings are aligned to avoid interference with the blade (130) so that the blade (130) and dilator tip can be advanced past the radially expanded wings.

4. A catheter as in claim 1, wherein the catheter body comprises an inner shaft connected to the dilator tip and an outer sheath retractable over the inner shaft, wherein the stent is self-expanding, and can be constrained in a non-expanded configuration between the shaft and sheath.

5. A catheter as in claim 4, wherein the outer sheath is coupled to the blade (130) so that the blade (130) can be distally advanced from the dilator tip and retracted into the dilator tip by advancing and retracting the outer sheath.

6. A catheter as in claim 1, wherein the blade (130) is slidably received in a groove in the needle (126).

7. A catheter as in claim 4, wherein a distal end of the outer sheath is provided with a plurality of slots arranged to receive the plurality of radially expandable wings of the anchor structure.

8. A catheter as in claim 1, wherein the blade (130) is configured to be advanceable from the dilator tip by the advancement of the needle (126) relative to the dilator tip.

9. A catheter as in claim 1, wherein the blade (130) is configured to be extendable from the dilator tip in both an axial and a radial direction.

## Patentansprüche

1. Katheter zum Schaffen eines Durchgangs durch gegenüberliegende luminale Wände und zur Stent-Abgabe, wobei der Katheter aufweist;
einen Katheterkörper (102), der ein proximales Ende (106), ein distales Ende (104) und einen zentralen Durchgang dort hindurch aufweist;
eine Dilatorspitze, die mit dem distalen Ende des Katheterkörpers (102) gekoppelt ist;
eine Nadel (126), die eine eindringende distale Spitze aufweist, wobei die Nadel (126) hin- und herbeweglich im zentralen Durchgang angebracht ist, so dass die distale Spitze über die Dilatorspitze des Katheterkörpers (102) hinaus vorgeschoben werden kann, um die gegenüberliegenden luminalen Wände zu durchdringen;
eine Ankerstruktur, die von der Nadel (126) einsetzbar ist und mehrere radial ausdehnbare Flügel aufweist, wobei die Ankerstruktur eingerichtet ist, ausgedehnt und mittels der Nadel (126) proximal gegen die gegenüberliegenden luminalen Wände gezogen zu werden;
eine Klinge (130), die von der Dilatorspitze ausfahrbar ist, um den Durchgang zu schneiden, wenn die Dilatorspitze durch den Durchgang vorgeschoben wird; und
einen radial ausdehnbaren Stent (134), der an einem distalen Bereich des Katheterkörpers (102) geführt wird.

2. Katheter nach Anspruch 1, wobei die Dilatorspitze mehrere Schlitze aufweist, die angeordnet sind, die radial ausgedehnten Flügel aufzunehmen, wenn die Dilatorspitze über die Nadel (126) vorgeschoben wird.

3. Katheter nach Anspruch 2, wobei die ausdehnbaren Flügel ausgerichtet sind, um eine Behinderung der Klinge (130) zu vermeiden, so dass die Klinge (130) und die Dilatorspitze über die radial ausgedehnten Flügel hinaus vorgeschoben werden können.

4. Katheter nach Anspruch 1, wobei der Katheterkörper einen inneren Schaft, der mit der Dilatorspitze verbunden ist, und eine äußere Hülle aufweist, die über den inneren Schaft zurückziehbar ist, wobei der Stent selbstausdehnend ist, und zwischen dem Schaft und der Hülle in einer nicht ausgedehnten Konfiguration eingezwängt werden kann.

5. Katheter nach Anspruch 4, wobei die äußere Hülle mit der Klinge (130) gekoppelt ist, so dass die Klinge (130) von der Dilatorspitze distal vorgeschoben und durch Vorschieben und Zurückziehen der äußeren Hülle in die Dilatorspitze eingezogen werden kann.

6. Katheter nach Anspruch 1, wobei die Klinge (130) verschiebbar in eine Nut in der Nadel (126) aufgenommen ist.

7. Katheter nach Anspruch 4, wobei ein distales Ende der äußeren Hülle mit mehreren Schlitzen versehen ist, die angeordnet sind, die mehreren radial ausdehnbaren Flügel der Ankerstruktur aufzunehmen.

8. Katheter nach Anspruch 1, wobei die Klinge (130) konfiguriert ist, durch das Vorschieben der Nadel (126) relativ zur Dilatorspitze von der Dilatorspitze vorschiebbar zu sein.

9. Katheter nach Anspruch 1, wobei die Klinge (130) konfiguriert ist, sowohl in eine axiale als auch eine radiale Richtung aus der Dilatorspitze ausfahrbar zu sein.

## Revendications

1. Cathéter permettant la création d'un passage au travers de parois luminales adjacentes et la pose d'un stent, ledit cathéter comprenant :
un corps de cathéter (102) ayant une extrémité proximale (106), une extrémité distale (104) et un passage central ;
une pointe de dilatation reliée à l'extrémité distale du corps de cathéter (102) ;
une aiguille (126) ayant une extrémité distale de pénétration, ladite aiguille (126) étant montée de manière à être mobile en va-et-vient dans le passage central pour permettre l'avancée de l'extrémité distale au-delà de la pointe de dilatation du corps de cathéter (102) afin de pénétrer dans les parois luminales adjacentes ;
une structure d'ancrage déployable depuis l'aiguille (126) et comprenant une pluralité de d'ailes radialement expansibles, ladite structure d'ancrage étant prévue pour être déployée et tirée proximalement par l'aiguille (126) contre les parois luminales adjacentes ;
une lame (130) pouvant s'étendre depuis la pointe de dilatation pour découper le passage quand la pointe de dilatation est avancée dans le passage ; et
un stent (134) radialement expansible supporté sur une zone distale du corps de cathéter (102).

2. Cathéter selon la revendication 1, où la pointe de dilatation présente une pluralité de fentes disposées pour recevoir les ailes radialement expansibles quand la pointe de dilatation est avancée sur l'aiguille (126).

3. Cathéter selon la revendication 2, où les ailes expansibles sont alignées pour empêcher une interférence avec la lame (130), de manière à permettre l'avancée de la lame (130) et de la pointe de dilatation au-delà des ailes radialement expansibles.

4. Cathéter selon la revendication 1, où le corps de cathéter comprend une tige intérieure raccordée à la pointe de dilatation et une gaine extérieure rétractable sur la tige intérieure, le stent étant auto-expansible et pouvant être retenu dans une configuration de non-expansion entre la tige et la gaine.

5. Cathéter selon la revendication 4, où la gaine extérieure est raccordée à la lame (130) de manière à permettre l'avancée distale de la lame (130) depuis la pointe de dilatation et son retrait dans la pointe de dilatation par avancée et rétraction de la gaine extérieure.

6. Cathéter selon la revendication 1, où la lame (130) est logée de manière coulissante dans une rainure de l'aiguille (126).

7. Cathéter selon la revendication 4, où une extrémité distale de la gaine extérieure est pourvue d'une pluralité de fentes disposées pour recevoir la pluralité d'ailes radialement expansibles de la structure d'ancrage.

8. Cathéter selon la revendication 1, où la lame (130) est prévue pour pouvoir être avancée depuis la pointe de dilatation par l'avancée de l'aiguille (126) par rapport à la pointe de dilatation.

9. Cathéter selon la revendication 1, où la lame (130) est prévue pour pouvoir s'étendre dans les directions axiale et radiale depuis la pointe de dilatation.
